# EUROPEAN PATENT APPLICATION

(11) **EP 4 591 846 A1**
(43) Date of publication of application: **30.07.2025**
(21) Application number: 24154398.2
(22) Date of filing: 29.01.2024
(51) Int. Cl.: A61K 6/62, A61K 6/887

(54) **DENTAL COMPOSITION**

(71) Applicant: DENTSPLY SIRONA Inc., York, PA 17401 (US); DENTSPLY DETREY GmbH, 78467 Konstanz (DE)
(72) Inventor: Fredrich, Sebastian, 78467 Konstanz (DE); Trötschler, Tobias, 78467 Konstanz (DE)
(74) Representative: Crow, Martin

(57) **Abstract**

The present invention relates to a polymerizable dental composition comprising a mixture of
(a) at least one polymerizable monomer having a polymerizable carbon-carbon double bond;
(b) an initiator system which is soluble in the mixture, comprising
(b1) a photoinitiator compound, and
(b2) a compound of the following formula (I), or a salt thereof: 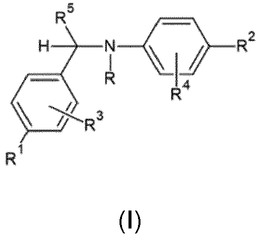 wherein
R¹ and R²,
which may be the same or different, independently represent a hydrogen atom, or a C₁₋₆ alkyl group;
R³ and R⁴
which may be the same or different, independently represent a hydrogen atom, or a C₁₋₆ alkyl group, or R³ and R⁴ together represent a single bond, a methylene group, or form together with the carbon atoms to which they are attached an 8- to 10-membered non-aromatic heterocyclic ring, provided that either R³ or R⁴ does not form a ring with R or R⁵;
R⁵
represents a hydrogen atom or a C₁₋₆ alkyl group, or R⁵ forms with either R³ or R⁴, respectively, and together with the carbon atoms to which they are attached a non-aromatic 6- to 10-membered heterocyclic ring, provided that R⁵ does not form a ring with R; and
R
represents a hydrogen atom, an optionally substituted phenyl group, or a C₁₋₆ alkyl group which may be substituted by an optionally substituted phenyl group, wherein the substituent on the optionally substituted phenyl group is selected from a C₁₋₆ alkyl group, or R forms together with either R³, R⁴ or R⁵, respectively, and the carbon atoms to which they are attached a non-aromatic 5- to 10-membered heterocyclic ring which may contain an endocyclic group selected from an amide group, an ester group, an ether group, or an amino group optionally substituted by a C₁₋₆ alkyl group, or R forms a C₁₋₃ alkylene bridge across the 8 to 10 membered heterocyclic ring formed by R³ and R⁴ and the carbon atoms to which they are attached.

## Description

### Field of the Invention

The present invention relates to a polymerizable dental composition comprising a specific polymerization initiator system containing a combination of a photoinitiator and a specific aromatic benzyl amine compound. The present invention also relates to the use of the polymerization initiator system in a photocurable dental composition. The aromatic benzyl amine compound is highly active as a coinitiator in a polymerization reaction of a monomer having a polymerizable carbon-carbon double bond in the presence of a photoinitiator while at the same time reducing the risk for adverse effects of the composition on the patient.

### Background of the Invention

Polymerizable dental compositions are known. Generic compositions contain polymerizable monomers, an initiator system and further components such as particulate fillers and solvents depending on the particular clinical use. Initiator systems may be activated chemically, thermally, or by irradiation. When the initiator system is adapted to be activated by irradiation only, the initiator system may be stored in admixture with the polymerizable monomers so that mixing of multiple components prior to use is avoided. During photocuring of a dental composition containing polymerizable monomers, the photoinitiator system generates free radicals upon exposure to visible light. Free radicals may be typically produced by either of two pathways:
(1) the photoinitiator compound undergoes excitation by energy absorption with subsequent decomposition of the compound into one or more radicals (Norrish type I), or
(2) the photoinitiator compound undergoes excitation and the excited photoinitiator compound interacts with a second compound by either energy transfer or a redox reaction to form free radicals from any of the compounds (Norrish type II).

In order to be useful in a clinical setting, the photocuring of a generic polymerizable dental composition must meet in combination a number of conditions:
- The rate of polymerization must be sufficiently high so that polymerization may be accomplished within about 10-40 seconds to avoid discomfort on the part of the patient.
- The spectrum and intensity of light source required for irradiation must not harm any hard or soft oral tissues of the patient.
- The heat of polymerization must be low to avoid damage of the pulp tissue via heat generated from absorption and conversion into thermal energy.
- Polymerization of the bulk of the restoration and the interface layer to the residual dental tissue must be complete since unreacted monomers can have an adverse effect on soft dental tissue.
- The components of the polymerizable dental composition should not cause any toxic, inflammatory, allergic, carcinogenic or mutagenic reactions in the patient.

In addition, photoinitiator systems often are required to have a high acid resistance, solubility, thermal stability, and storage stability when incorporated into a dental composition.

Finally, given that dental compositions usually contain (meth)acrylate or (meth)acrylamide monomers, free radical photocuring may be inhibited by the presence of oxygen. Oxygen inhibition is due to the rapid reaction of propagating radicals with oxygen molecules to yield peroxyl radicals which are not as reactive towards carbon-carbon unsaturated double bonds and therefore do not initiate or participate in any photopolymerization reaction. Oxygen inhibition may lead to premature chain termination and, therefore, incomplete photocuring. Nevertheless, a certain degree of oxygen inhibition on the top surface of the adhesive layer is required for the bonding to the adjacent restorative.

Despite great efforts of researchers in the field, the number of effective and safe photoinitiator systems for polymerizable dental compositions is limited. In fact, a great number of dental compositions rely on a combination of camphorquinone (CQ) and aromatic amines such as N,N-dimethyl-p-toluidine or ethyl 4-(dimethylamino)benzoate. CQ as such can photoinitiate polymerization only at a low rate whereby the aromatic amine is required as a coinitiator for accelerating the polymerization. It is assumed that the formation of aminoalkyl radicals occurs via photoinduced electron transfer whereby the interaction of CQ with the coinitiator forms an aminoalkyl radical which initiates the polymerization of the polymerizable monomers. For this purpose, the reactive aminoalkyl radical must approach the unsaturated bond in the monomer.

Although aromatic amines such as N,N-dimethyl-p-toluidine or ethyl 4-(dimethylamino)benzoate are advantageous in promoting polymerization, the compound gives rise to concerns regarding the safety of the patient.

It is the problem of the present invention to provide a polymerizable dental composition wherein the components, notably the coinitiator does not give rise to concerns regarding the safety of the patient while at the same time the initiator system provides a rate of polymerization so that polymerization may be accomplished in a reasonably short time, the spectrum and intensity of light source required for irradiation does not harm any hard or soft oral tissues of the patient, the heat of polymerization is low to avoid damage of the pulp tissue via heat conversion in thin layers, and polymerization of the inner part of the restoration is complete and unreacted monomers are avoided.

### Summary of the Invention

The present invention provides a polymerizable dental composition comprising a mixture of
(a) at least one polymerizable monomer having a polymerizable carbon-carbon double bond;
(b) an initiator system which is soluble in the mixture, comprising
   (b1) a photoinitiator compound, and
   (b2) a compound of the following formula (I), or a salt thereof: 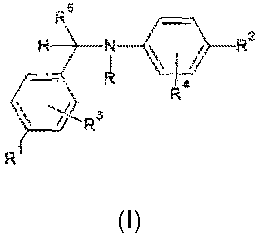 wherein
      - R¹ and R²: , which may be the same or different, independently represent a hydrogen atom, or a C₁₋₆ alkyl group;
      - R³ and R⁴: which may be the same or different, independently represent a hydrogen atom, or a C₁₋₆ alkyl group, or
      R³ and R⁴ together represent a single bond, a methylene group, or form together with the carbon atoms to which they are attached an 8- to 10-membered non-aromatic heterocyclic ring, provided that either R³ or R⁴ does not form a ring with R or R⁵;
      - R⁵: represents a hydrogen atom or a C₁₋₆ alkyl group, or
      R⁵ forms with either R³ or R⁴, respectively, and together with the carbon atoms to which they are attached a non-aromatic 6-to 10-membered heterocyclic ring, provided that R⁵ does not form a ring with R; and
      - R: represents a hydrogen atom, an optionally substituted phenyl group, or a C₁₋₆ alkyl group which may be substituted by an optionally substituted phenyl group, wherein the substituent on the optionally substituted phenyl group is selected from a C₁₋₆ alkyl group, or
      R forms together with either R³, R⁴ or R⁵, respectively, and the carbon atoms to which they are attached a non-aromatic 5- to 10-membered heterocyclic ring which may contain an endocyclic group selected from an amide group, an ester group, an ether group, or an amino group optionally substituted by a C₁₋₆ alkyl group, or
      R forms a C₁₋₃ alkylene bridge across the 8 to 10 membered heterocyclic ring formed by R³ and R⁴ and the carbon atoms to which they are attached.

The present invention is based on the recognition that a compound of formula (I) in combination with a photosensitizer such as CQ provides a rate of polymerization which is sufficiently high so that polymerization of a polymerizable dental composition may be accomplished within at most 10-40 seconds while the irradiation spectrum and intensity of a conventional light source may be used which is considered to be safe for hard or soft oral tissues. Moreover, the heat of polymerization may be controlled by using a compound of formula (I) to avoid damage of the pulp tissue via heat conversion in thin layers. Furthermore, the use of a compound of formula (I) provides high conversion so that the polymerization of the inner part of the restoration is essentially complete. Finally, a compound of formula (I) is not expected to cause any toxic, inflammatory, allergic, carcinogenic or mutagenic reactions in the patient.

The present invention also provides a use of a compound of the following formula (I), or a salt thereof: 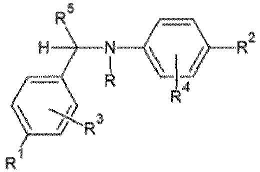 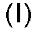 wherein R, and R¹ to R⁵ are as defined above, in a photocurable dental composition.

A polymerizable dental composition according to the present invention contains a compound of formula (I) as a coinitiator which is highly effective, and does not result in toxic, inflammatory, allergic, carcinogenic or mutagenic reactions in a patient.

### Brief Description of the Figures

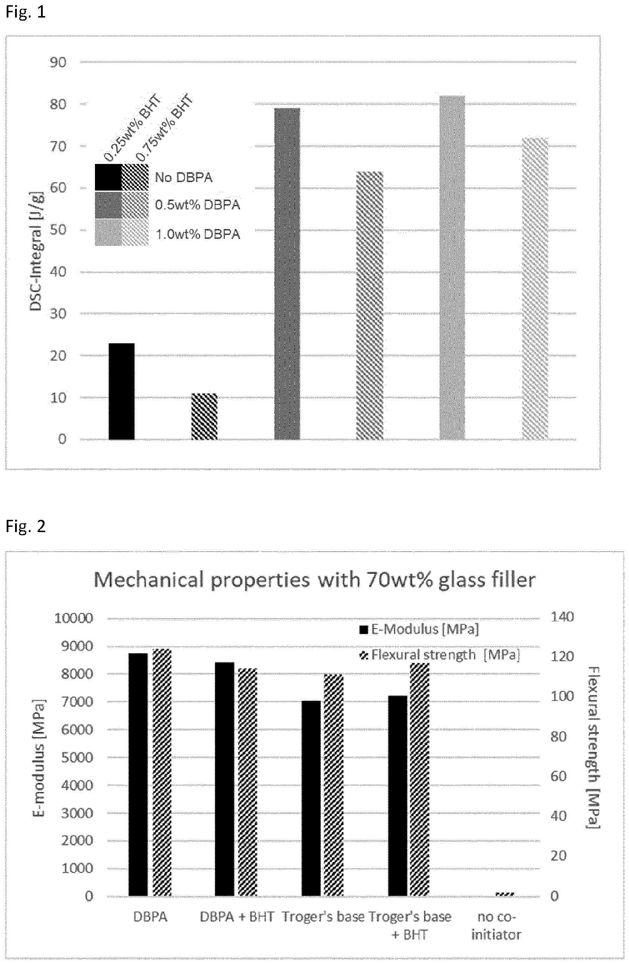

Fig. 1 depicts the polymerization heat obtained from dynamic scanning calorimetry (DSC) indicating the conversion of the double bonds upon irradiation with a defined intensity and time. The six mixtures consist of UDMA-resin with 0.25 wt% camphorquinone and various amounts of dibenzyl aniline with different amounts of BHT. The results show that increasing amounts of co-initiator dibenzyl aniline cause higher conversion of double bonds. Due to the urethane function of UDMA-resin, the blank sample also shows considerable polymerization. The inhibitor BHT has only a minor suppressing effect on the photoinitiation.
Fig. 2 depicts the mechanical properties obtained from a three-point bending test from pastes containing 1 wt% dibenzyl aniline (DBPA) or Troger's base in BisGMA/TEGDMA 70:30 and additional 70 wt% silanized glass. All mixtures show high values especially in comparison with a sample just containing camphorquinone without co-initiator. The addition of 0.25 wt% BHT has only a minor effect.

### Detailed Description of the Preferred Embodiments

The terms "polymerization" or "polymerizable" relate to the combining by covalent bonding of many smaller molecules, such as monomers in the form of radical-polymerizable or cationically polymerizable compounds, to form larger molecules, that is, macromolecules or polymers. The monomers may be combined to form only linear macromolecules or they may be combined to form three-dimensional macromolecule, commonly referred to as crosslinked polymers. In case of a higher conversion rate of the polymerizable monomer, the amount of multifunctional monomers may be reduced or the leaching problem may be alleviated.

The term "photocurable" refers to a dental composition that will radically and/or cationically polymerize into a crosslinked polymer network when irradiated for example with actinic radiation such as ultraviolet (UV), visible, or infrared radiation. "Actinic radiation" is any electromagnetic radiation that is capable of producing photochemical action and can have a wavelength of at least 150 nm and up to and including 1250 nm, and typically at least 300 nm and up to and including 750 nm.

The term "radical-polymerizable" as used herein in connection with compounds (a) means any compound capable of radical polymerization. Typically, compounds (a) are radical-polymerizable due to a polymerizable double bond, preferably one or more carbon-carbon double bonds. Examples of the polymerizable double bond include vinyl, conjugated vinyl, allyl, acryl, methacryl and styryl. More preferably, the polymerizable double bond is selected from the group consisting of acryl, methacryl and styryl. Acryl and methacryl may be (meth)acryloyl or (meth)acrylamide. Most preferably, for compounds (a), the polymerizable double bound is acryl or methacryl and/or acrylamide or methacrylamide.

The term "cationically polymerizable" as used herein in connection with compounds (a) means any compound capable of cationic polymerization. Typically, compounds (a) are cationically polymerizable due to the presence of a functional group having a carbon-carbon double bond such as a vinyl ether group.

The term "polymerization initiator system" refers to a system comprising (i) a photoinitiator compound and (ii) a coinitiator compound. Optionally, the polymerization initiator system may further comprise (iii) an additional electron-donor.

The term "photoinitiator" used in connection with the compound (i) refers to a compound that is activated, for example by forming free radicals, typically by exposure to light or interaction with another compound such as coinitiator compound (ii) and/or electron-donor (iii) in a photochemical process.

The present invention relates to a polymerizable, notably a photocurable dental composition. The photocurable dental composition may be a dental composite, a dental adhesive, a dental glass ionomer cement, or a dental impression material.

### The polymerizable compounds (a)

The photocurable dental composition comprises (a) one or more polymerizable compounds. The compounds may be radical-polymerizable or cationically polymerizable.

The one or more radical-polymerizable compounds (a) may preferably be radical-polymerizable N-substituted alkyl acrylic or acrylic acid amide monomers or a (meth)acrylate compounds.

A radical-polymerizable N-substituted alkyl acrylic or acrylic acid amide monomer may be preferably selected from compounds characterized by one of the following formulae (X), (XI) and (XII):

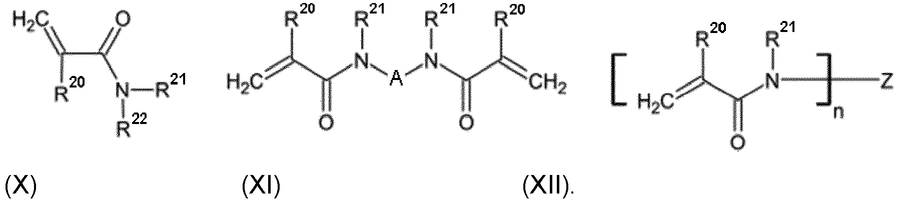

In formulae (X), (XI) and (XII), R²⁰, R²¹ and R²² independently represent a hydrogen atom or a C1 to C8 alkyl group; A represents a divalent substituted or unsubstituted organic residue having from 1 to 10 carbon atoms, whereby said organic residue may contain from 1 to 3 oxygen and/or nitrogen atoms; Z represents a saturated at least trivalent substituted or unsubstituted C1 to C8 hydrocarbon group, a saturated at least trivalent substituted or unsubstituted cyclic C3 to C8 hydrocarbon group, and n is at least 3.

Preferably, the one or more radical-polymerizable compounds (a) include bisacrylamides such as N,N'-diethyl-1,3-bisacrylamido-propan (BADEP), 1,3-bisacrylamido-propan (BAP), 1,3-bisacrylamido-2-ethyl-propan (BAPEN), N,N'-(2E)-2-butene-1,4-diylbis[N-2-propen-1-yl-2-propenamide] (BAABE) , N,N-di(cyclopropyl acrylamido) propane (BCPBAP) and N,N'-(2-hydroxy-1,3-propanediyl)bis[N-2-propen-1-yl-2-propenamide] (DAAHP).

Alternatively or additionally, for the one or more radical-polymerizable compounds (a), a (meth)acrylate compound may be selected from the group of methyl acrylate, methyl methacrylate, ethyl acrylate, ethyl methacrylate, propyl acrylate, propyl methacrylate, isopropyl acrylate, isopropyl methacrylate, 2-hydroxyethyl acrylate, 2-hydroxyethyl methacrylate (HEMA), hydroxypropyl acrylate, hydroxypropyl methacrylate, tetrahydrofurfuryl acrylate, tetrahydrofurfuryl methacrylate, glycidyl acrylate, glycidyl methacrylate, the diglycidyl methacrylate of bis-phenol A ("bis-GMA"), glycerol mono-and di- acrylate, glycerol mono- and dimethacrylate, ethyleneglycol diacrylate, ethyleneglycol dimethacrylate, polyethyleneglycol diacrylate (where the number of repeating ethylene oxide units vary from 2 to 30), polyethyleneglycol dimethacrylate (where the number of repeating ethylene oxide units vary from 2 to 30 especially triethylene glycol dimethacrylate ("TEGDMA"), neopentyl glycol diacrylate, neopentylglycol dimethacrylate, trimethylolpropane triacrylate, trimethylol propane trimethacrylate, mono-, di-, tri-, and tetra- acrylates and methacrylates of pentaerythritol and dipentaerythritol, 1,3-butanediol diacrylate, 1,3-butanediol dimethacrylate, 1,4-butanedioldiacrylate, 1,4-butanediol dimethacrylate, 1,6-hexane diol diacrylate, 1,6-hexanediol dimethacrylate, di-2-methacryloyloxethyl hexamethylene dicarbamate, di-2-methacryloyloxyethyl trimethylhexanethylene dicarbamate, di-2-methacryloyl oxyethyl dimethylbenzene dicarbamate, methylene-bis-2-methacryloxyethyl-4-cyclohexyl carbamate, di-2-methacryloxyethyl-dimethylcyclohexane dicarbamate, methylene-bis-2-methacryloxyethyl-4-cyclohexyl carbamate, di-1-methyl-2-methacryloxyethyl-trimethyl-hexamethylene dicarbamate, di-1-methyl-2-methacryloxyethyl-dimethylbenzene dicarbamate, di-1-methyl-2-methacryloxyethyl-dimethylcyclohexane dicarbamate, methylene-bis-1-methyl-2-methacryloxyethyl-4-cyclohexyl carbamate, di-1-chloromethyl-2-methacryloxyethyl-hexamethylene dicarbamate, di-1-chloromethyl-2-methacryloxyethyl-trimethylhexamethylene dicarbamate, di-1-chloromethyl-2-methacryloxyethyl-dimethylbenzene dicarbamate, di-1-chloromethyl-2-methacryloxyethyl-dimethylcyclohexane dicarbamate, methylene-bis-2-methacryloxyethyl-4-cyclohexyl carbamate, di-1-methyl-2-methacryloxyethyl-hexamethylene dicarbamate, di-1-methyl-2-methacryloxyethyl-trimethylhexamethylene dicarbamate, di-1-methyl-2-methacryloxyethyl-dimethylbenzene dicarbamate, di-1-methyl-2-metha-cryloxyethyl-dimethylcyclohexane dicarbamate, methylene-bis-1-methyl-2-methacryloxyethyl-4-cyclohexyl carbamate, di-1-chloromethyl-2-methacryloxyethyl-hexamethylene dicarbamate, di-1-chloromethyl-2-methacryloxyethyl-trimethylhexamethylene dicarbamate, di-1-chloromethyl-2-methacryloxyethyl-dimethylbenzene dicarbamate, di-1-chloromethyl-2-methacryloxyethyl-dimethylcyclohexane dicarbamate, methylene-bis-1-chloromethyl-2-methacryloxyethyl4-cyclohexyl carbamate, 2,2'-bis(4-methacryloxyphenyl)propane, 2,2'bis(4-acryloxyphenyl)propane, 2,2'-bis[4(2-hydroxy-3-methacryloxy-phenyl)]propane, 2,2'-bis[4(2-hydroxy-3-acryloxy-phenyl)propane, 2,2'-bis(4-methacryloxyethoxyphenyl)propane, 2,2'-bis(4-acryloxyethoxyphenyl)propane, 2,2'-bis(4-methacryloxypropoxyphenyl)propane, 2,2'-bis(4-acryloxypropoxyphenyl)propane, 2,2'-bis(4-methacryloxydiethoxyphenyl)propane, 2,2'-bis(4-acryloxydiethoxyphenyl)propane, 2,2'-bis[3(4-phenoxy)-2-hydroxypropane-1-methacrylate]propane,and 2,2'-bis[3(4-phenoxy)-2-hydroxypropane-1-acryalte]propane, may be mentioned. Other suitable examples of radical-polymerizable components are isopropenyl oxazoline, vinyl azalactone, vinyl pyrrolidone, styrene, divinylbenzene, urethane acrylates or methacrylates, epoxy acrylates or methacrylates and polyol acrylates or methacrylates.

Preferably, the one or more radical-polymerizable compounds (a) contain one or two radical-polymerizable groups, more preferably two radical-polymerizable groups, such as acrylamides or bisacrylamides like Bis-GMA, TEGDMA, UDMA, BADEP, BAP, BAPEN, BAABE, BCPBAP and DAAHP.

It is preferable that a blending ratio of the one or more radical-polymerizable compounds (a) to the entire photocurable dental composition is 5 to 80% by weight. More preferably, the blending ratio is 10 to 60% by weight.

### The polymerization initiator system

The polymerizable dental composition further comprises a polymerization initiator system which is soluble in the mixture. Specifically, the polymerization initiator may be fully dissolved in the required amount in the photocurable dental composition. Typically, the polymerizable dental composition contains 0.01 to 20 percent by weight, more preferably 0.1 to 10 percent by weight based on the total amount of the composition of the polymerization initiator system.

The polymerization initiator system comprises a photoinitiator compound (b1). Preferably, the photoinitiator compound has a light absorption maximum in the range from 300 to 800 nm, in particular 350 to 500 nm. The polymerization initiator system (b) may comprise one or a mixture of two or more photoinitiator compound(s) (b1). The photoinitiator compound may be an acylphosphine oxide or a 1,2-diketone. Suitable 1,2-diketone photoinitiator compounds (b1) may be selected from the group consisting of camphorquinone, benzil, 2,2'-3 3'- and 4,4'-dihydroxylbenzil, 2,3-butanedione, 2,3-pentanedione, 2,3-hexanedione, 3,4-hexanedione, 2,3-heptanedione, 3,4-heptanedione, 2,3-octanedione, 4,5-octanedionefuril, 1,2-cyclohexanedione, 1,2-naphthaquinone, and acenaphthaquinone. Camphorquinone is preferred.

Typically, the polymerizable dental composition contains 0.005 to 5 percent by weight, more preferably 0.05 to 3 percent by weight based on the total amount of the composition of the photoinitiator compound (b1).

The initiator system further comprises a compound (b2) of the following formula (I), or a salt thereof:

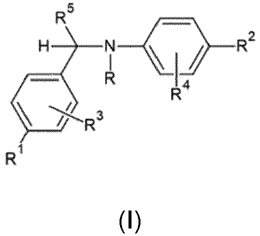

In formula (I), R¹ and R² may be the same or different. R¹ and R² independently represent a hydrogen atom, or a C₁₋₆ alkyl group. According to the invention, a C₁₋₆ alkyl group can include straight or branched alkyl groups having 1 to 6 carbon atoms, preferably 1 to 4 carbon atoms, for example, methyl, ethyl, n-propyl, isopropyl, n-butyl, isobutyl, sec-butyl, tert-butyl, n-pentyl, isopentyl and n-hexyl. According to further preferred embodiments, R¹ and R² are both hydrogen atoms or R¹ and R² are both methyl groups.

In formula (I), R³ and R⁴ may be the same or different. R³ and R⁴ independently may represent a hydrogen atom, or a C₁₋₆ alkyl group. The C₁₋₆ alkyl group may include straight, or branched alkyl groups as defined for R¹ and R². According to a preferred embodiment, R³ and R⁴ represent hydrogen atoms or methyl groups, in particular a hydrogen atom.

Alternatively, R³ and R⁴ together represent a single bond, a methylene group, or form together with the carbon atoms to which they are attached an 8- to 10-membered non-aromatic heterocyclic ring, provided that either R³ or R⁴ does not form a ring with R or R⁵.

When R³ and R⁴ form single bond linking the ortho positions of the aromatic rings, preferably a compound of the following formula (I-a), or a salt thereof, is formed: 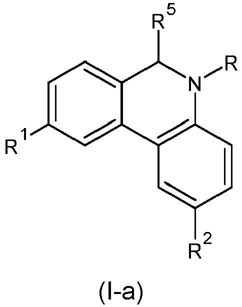 wherein R, R¹, R² and R⁵ are as defined above.

According to a specific embodiment of a compound of formula (I-a), R and R⁵ and the carbon atoms to which they are attached may form a non-aromatic 5- to 10-membered heterocyclic ring which may contain an endocyclic group selected from an amide group, an ester group, an ether group, or an amino group optionally substituted by a C₁₋₆ alkyl group, for example a compound of the following formula (1-a1), or a salt thereof: 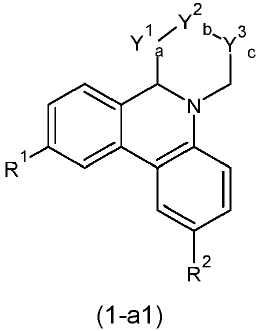 wherein R¹ and R² are as defined above, Y¹, Y², and Y³ are methylene groups, a, b and c are independently selected from 0 or an integer of from 1 to 7, whereby one of Y¹, Y², and Y³ may be an endocyclic group selected from an amide group, an ester group, an ether group, or an amino group optionally substituted by a C₁₋₆ alkyl group, so that a non-aromatic 5- to 10-membered heterocyclic ring is formed.

When R³ and R⁴ form a methylene group linking the ortho positions of the aromatic rings, preferably a compound of the following formula (I-b), or a salt thereof, is formed: 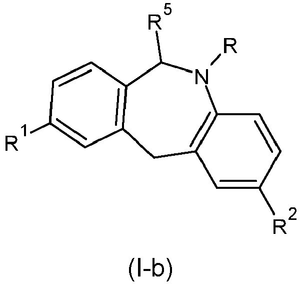 wherein R, R¹, R² and R⁵ are as defined above.

According to a specific embodiment of a compound of formula (1-a), R and R⁵ and the carbon atoms to which they are attached may form a non-aromatic 5- to 10-membered heterocyclic ring which may contain an endocyclic group selected from an amide group, an ester group, an ether group, or an amino group optionally substituted by a C₁₋₆ alkyl group, for example a compound of the following formula (1-b1), or a salt thereof: 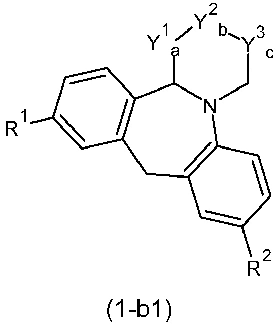 wherein R¹ and R² are as defined above, Y¹, Y², and Y³ are methylene groups, a, b and c are independently selected from 0 or an integer of from 1 to 7, whereby one of Y¹, Y², and Y³ may be an endocyclic group selected from an amide group, an ester group, an ether group, or an amino group optionally substituted by a C₁₋₆ alkyl group, so that a non-aromatic 5- to 10-membered heterocyclic ring is formed.

According to a further preferred embodiment of formula (1-b1), the non-aromatic heterocyclic ring contains an endocyclic amino group optionally substituted by a C₁₋₆ alkyl group of the following formula: 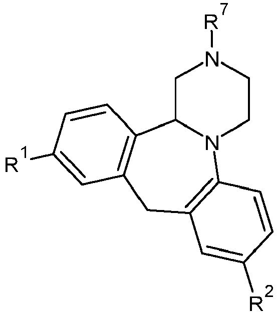 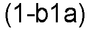 wherein R¹ and R² are as defined above, and R⁷ is a C₁₋₆ alkyl group.

According to a further preferred embodiment, a compound of formula (1-b1a) is mianserin, wherein R¹ and R² are hydrogen atoms, and R⁷ is a methyl group of the following formula:

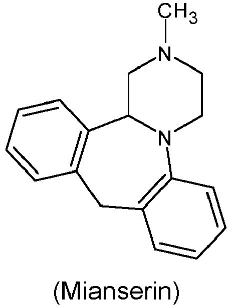

According to a preferred embodiment, R³ and R⁴ are attached in the ortho position of a compound of formula (I), respectively, and together represent a group of the following formula (II): 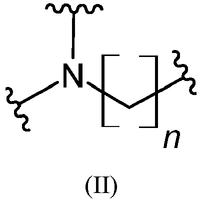 wherein n is an integer of from 1 to 3, and R forms a divalent bridge across the 8 to 10 membered non-aromatic heterocyclic ring formed by R³ and R⁴ and the carbon atoms to which they are attached.

A particularly preferred compound of formula (I) is a compound of the following formula (I-c), or a salt thereof: 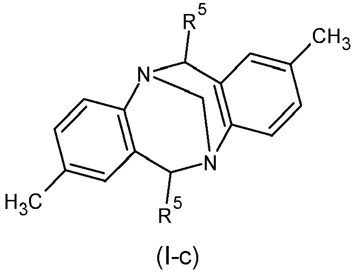 wherein R⁵ is a hydrogen atom or a C₁₋₆ alkyl group. When R⁵ in formula (I-c) is a hydrogen atom, the compound is Troger's Base, which is a particularly preferred compound. Alternatively, either R³ or R⁴ may form a ring with R or R⁵ as further described below.

In formula (I), R⁵ may represent a hydrogen atom or a C₁₋₆ alkyl group. Preferably, R⁵ is a hydrogen atom.

Alternatively, R⁵ may form with either R³ or R⁴, respectively, and together with the carbon atoms to which they are attached a non-aromatic 6- to 10-membered heterocyclic ring.

A corresponding compound having an alicyclic ring formed by R³ and R⁵ may be a compound of the following formula (I-d1): 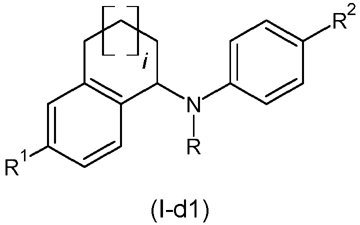 wherein R¹, R², and R are as defined above, and i is an integer of from 1 to 4.

A corresponding compound having an alicyclic ring formed by R⁴ and R⁵ may be a compound of the following formula (I-d2): 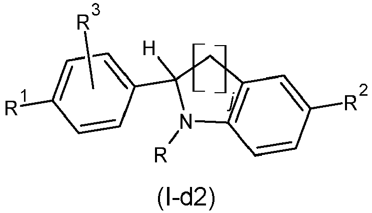 wherein R¹, R², R³, and R are as defined above, and j is an integer of from 2 to 6.

In formula (I), R may represent a hydrogen atom, an optionally substituted phenyl group, or a C₁₋₆ alkyl group which may be substituted by an optionally substituted phenyl group, wherein the substituent on the optionally substituted phenyl group is selected from a C₁₋₆ alkyl group. According to a preferred embodiment, R represents a methyl group, or a benzyl group which may be substituted by a C₁₋₆ alkyl group. More preferably, R is a methyl group or a benzyl group. In particular, a compound of formula (I) may be N-benzyl-N-methyl aniline or dibenzyl aniline.

Alternatively, R may form together with either R³, R⁴ or R⁵, respectively, and the carbon atoms to which they are attached a non-aromatic 5- to 10-membered heterocyclic ring which may contain an endocyclic group selected from an amide group, an ester group, an ether group, or an amino group optionally substituted by a C₁₋₆ alkyl group.

When R forms with R³ and together with the carbon atoms to which they are attached a non-aromatic 5- to 10-membered heterocyclic ring, a compound of the following formula (I-e1) is formed: 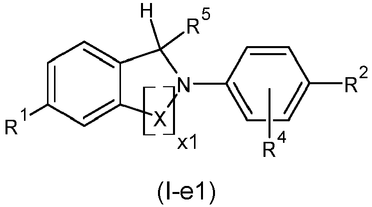 wherein R¹, R², R⁴, and R⁵ are as defined above, X is a methylene group, and when more than one X is present, one of the X may be an endocyclic group selected from an amide group, an ester group, an ether group, or an amino group optionally substituted by a C₁₋₆ alkyl group, and x1 is selected from an integer of from 1 to 5 so that 5- to 10-membered heterocyclic ring is formed.

When R forms with R⁴ and together with the carbon atoms to which they are attached a non-aromatic 5- to 10-membered non-aromatic heterocyclic ring, a compound of the following formula (I-e2) is formed: 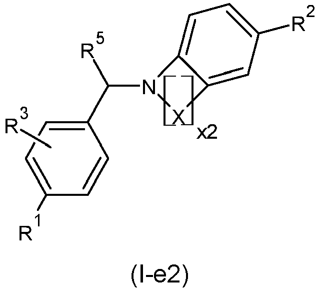 wherein R¹, R², R³, and R⁵ are as defined above, X is represent methylene groups, whereby one of the X may be an endocyclic group selected from an amide group, an ester group, an ether group, or an amino group optionally substituted by a C₁₋₆ alkyl group, and x2 is selected from an integer of from 2 to 5 so that 5- to 10-membered heterocyclic ring is formed.

Preferably, the compound of formula (I) is N-benzyl-N-methyl aniline, mianserin, Troger's base, dibenzyl aniline or 4-benzyl-1,3-dihydroquinoxalin-2-one.

Typically, the polymerizable dental composition contains 0.005 to 10 percent by weight, more preferably 0.05 to 5 percent by weight, still more preferably 0.1 to 3 percent by weight based on the total amount of the composition of the compound (b2) of formula (I).

Optionally, the polymerizable dental composition contains 0.005 to 5 percent by weight, more preferably 0.05 to 3 percent by weight, still more preferably 0.1 to 2 percent by weight based on the total amount of the composition of an additional accelerator such as a diphenyl iodonium salt (DPI).

Compounds of formula (I) may be accessed by the skilled person commercially, such as mianserin and Troger's Base. Alternatively, compounds of formula (I) may be prepared or by using synthetic methods described in the prior art which may be modified based on general knowledge to prepare any compound covered by formula (I).

Arylbenzalamines may be prepared according to US-A 5,536,877. Also, dibenzylaniline may be prepared using a mixture of dibutyl tin dichloride and dibutyl stannane with N-benzilideneaniline along with hexamethylphosphoric triamide dissolved in tetrahydrofuran which yields a tin amide compound. This then reacts with benzyl bromide to yield dibenzylaniline according to Fürstner, Alois (2014). Science of Synthesis Knowledge Updates 2013. Georg Thieme Verlag. pp. 72-73. ISBN 978-3-13-178881-8. N-benzyl-N-methyl aniline may also be prepared as described in Zarif M. et al. Can. J. Chern., 52,293 (1974).

Troger's Base may be prepared by p-toluidine and formaldehyde in an acidic solution based on Julius Tröger (1887). "Ueber einige mittelst nascirenden Formaldehydes entstehende Basen". Journal für Praktische Chemie. 36 (1): 225-245. Doi:10.1002/prac.18870360123. Alternatively, a synthesis may be based on Masa, Thierry; Pardo, Carmen; Elguero, José (2004). "A shorter synthesis of symmetrical 2,11-dimethyl-bis-Tröger's bases. A new molecular tweezer". Arkivoc. (EM-973K). Suitable substitution and/or mixtures of the p-toluidine precursor gives access to any derivatives of Troger's Base covered by formula (I).

Mianserin and derivatives thereof a may be obtained commercially or prepared in line with Sacre S. et al. Front. Immunol., 24 May 2019; Sec. Inflammation Volume 10 - 2019 | https://doi.org/10.3389/fimmu.2019.01167.

### Further components

Optionally, the photocurable dental composition of the present invention may further comprise a solvent and/or a particulate filler.

Suitable solvents may be selected from water, DMSO, alcohols such as methanol, ethanol, propanol (n-, iso-), butanol (n-, iso-, tert-), ketones such as acetone or the like.

The polymerizable dental composition of the present invention may preferably comprise 5 to 75 percent by weight based on the total weight of the composition of a solvent.

Suitable particulate fillers may be selected from fillers currently used in dental compositions. The filler should be finely divided and preferably has a maximum particle diameter less than about 100 µm and an average particle diameter less than about 10 µm. The filler may have a unimodal or polymodal (e.g., bimodal) particle size distribution.

The filler can be an inorganic material. It can also be a crosslinked organic material that is insoluble in the one or more radical-polymerizable compounds (a), and is optionally filled with inorganic filler. The filler can be radioopaque. Examples of suitable particulate inorganic fillers are naturally-occurring or synthetic materials such as quartz, nitrides such as silicon nitride, glasses derived from, for example Ce, Sb, Sn, Zr, Sr, Ba and Al, colloidal silica, feldspar, borosilicate glass, kaolin, talc, titania, and zinc glass, and submicron silica particles such as pyrogenic silicas. Examples of suitable non-reactive organic filler particles include filled or unfilled pulverized polycarbonates or polyepoxides. Preferably the surface of the filler particles is treated with a coupling agent in order to enhance the bond between the filler and the matrix. The use of suitable coupling agents include gamma-methacryloxypropyltrimethoxysilane, gamma-mercaptopropyltriethoxysilane, gamma-aminopropyltrimethoxysilane, and the like.

The particulate filler may also be a hybrid organic/inorganic filler, for example a SphereTEC^{®} filler obtainable by a process for the preparation of composite filler particles, comprising:
a) coating a particulate filler having a median particle size (D50) of from 1 to 1200 nm with a coating composition containing a film-forming agent forming a coating layer on the surface of the particulate filler, said coating layer displaying reactive groups on the surface of the coating layer, said reactive groups being selected from addition polymerizable groups and step-growth polymerizable groups, thereby forming a coated particulate filler; subsequently or concurrently
b) agglomerating the coated particulate filler, optionally in the presence of a further crosslinking agent and optionally in the presence of a further particulate filler not displaying reactive groups, for providing a granulation of the coated particulate filler wherein the granulation contains the coated particulate filler particles and the optional further particulate filler particles separated from and connected to each other by at least one coating layer, whereby the at least one coating layer may be crosslinked by crosslinking groups obtained by reacting the reactive groups and optionally a further crosslinking agent;
c) optionally milling, classifying and/or sieving the granulation of the coated particulate filler; and
d) optionally further crosslinking the granulation of the coated particulate filler; for providing composite filler particles having a median particle size (D50) of from 1 to 70 µm, wherein reactive groups are transformed into crosslinking groups obtained by reacting reactive groups and optionally a further crosslinking agent, and wherein the particulate filler is the main component by volume of the composite filler particles as further described in EP-A 2 604 247 and EP-A 3 984 516 .

Furthermore, the filler may be a glass filler. The glass filler is preferably a silanated glass filler. The surface of the glass may be modified by using a surface modifying agent. The surface modifying agent contains a modifying compound capable of reacting with surface atoms of the filler, thereby forming a covalent bond between the surface atoms of the filler and the modifying compound. Additionally, the modifying compound may contain one or more polymerizable double bonds reactive in the crosslinking reaction after the filler is modified. The modifying agent may contain one or more modifying compounds. Preferably, the modifying compound provides a polymerizable ligand capable of crosslinking which may be a compound of one of the following formulae (I), (II) and (III), or a hydrolysis product thereof.

Xᵣ R⁸₃₋ᵣSiL (I)

Xᵣ R⁸₂₋ᵣSiL'L" (II)

XᵣSiL'L"L‴ (III)

wherein
X represents a hydrolyzable group;
R⁸ represents an alkyl, cycloalkyl, cycloalkylalkyl, aralkyl or aryl group, L, L', L", and L‴
which may be the same or different represent independent from each other an organic group containing one or more polymerizable double bonds;
r is an integer of 1 to 3,
whereby the sum of X, R⁸, L, L', L", and L‴ is 4 for each of formula (I), (II), and (III).

Preferably, X is a halogen atom or OR⁹, wherein R⁹ is an alkyl, cycloalkyl, cycloalkylalkyl, aralkyl or aryl group. More preferably, R⁸ or R⁹ are independently an alkyl group.

In order to impart crosslinking capability to the organofunctional silicon compound, L, L', L", and L‴ contain one or more polymerizable double bonds capable of taking part in a crosslinking reaction. In a preferred embodiment, L, L', L", and L‴ may be selected from the group of allyl, (meth)acrylic ester groups, and (meth)acrylic amide groups.

The polymerizable dental composition of the present invention may preferably comprise 0.1 to 85 percent by weight based on the total weight of the composition of particulate filler.

The polymerizable dental compositions of the present invention may further contain stabilizers, pigments, free radical scavengers, polymerization inhibitors, reactive and nonreactive diluents, coupling agents to enhance reactivity of fillers, rheology modifiers, and surfactants.

Suitable stabilizers may be selected from reducing agents such as vitamin C, inorganic sulfides and polysulfides and the like.

Suitable polymerization inhibitors may be selected from BHT, hydroquinone monomethylether, 2,6-di-tert-butyl-p-cresol, tetramethyl piperidine N-oxyl radical, galvinoxyl radical, or polymerization inhibitors disclosed in EP-A 1776943. The polymerizable dental composition of the present invention may preferably comprise 0.001 to 5 percent by weight more preferably 0.01 to 2 percent by weight based on the total weight of the composition of the polymerization inhibitor.

Suitable pigment may be selected from the group comprising iron oxide, titanium oxide, barium sulfate, manganese oxide, zinc oxide, bismuth oxide and organic fluorophores.

In the preferred embodiment of the present invention, the dental composition comprises 10 to 99.5% by weight of the at least one polymerizable monomer, 0.1 - 3% by weight of the at least one polymerization initiator 0.05 to 5% by weight of the at least one polymerization coinitiator which is a compound of formula (I), 0.001-3.0% by weight of the at least one polymerization inhibitor and 0-85% by weight of the at least one filler.

### One-part or multi-part composition

The polymerizable dental composition according to the present invention may be a one-part or a multi-part polymerizable dental composition.

The term "one-part" as used herein means that all components of the polymerizable dental composition are comprised in one single part.

The term "multi-part" as used herein means that the components of the polymerizable dental composition are comprised in a multitude of separate parts. For example, a first part of components is comprised in a first part, while as second part of components is comprised in a second part, a third part of components may be comprised in a third part, a fourth part of components may be comprised in a fourth part, and so on.

Preferably, the polymerizable dental composition is a one-part or a two-part polymerizable dental composition, most preferably a one-part polymerizable dental composition.

### Use of the present polymerization initiator system (ii)

The compound of formula (I) or a salt thereof as described above may be used in a photocurable dental composition, preferably a polymerizable dental composition as described above. The compound of formula (I) is preferably part of an initiator system as described herein.

The present invention will now be further explained with the following examples and comparative examples.

### Examples

The following abbreviations and terms are used in the current specification:
UDMA= Urethane dimethacrylate resin
DMABE = N,N-Dimethylamino ethyl benzoate
DPI = Dimethyl diphenyl iodonium hexafluorophosphate
EBPADMA = Ethoxylated bisphenol A dimethacrylate
BAABE = N,N'-diallyl-1,4- bisacrylamido-(2E)-but-2-ene
BADEP = N,N'-diethyl-1,3-bisacrylamido-propane
CQ = Camphorquinone
Spectrum TPH = Spectrum Total Performance Hybrid
BHT = Butylated hydroxytoluene
Paste = A dental composition comprising a filler
TEGDMA = Triethylenglycol dimethacrylate
BisEMA = [2-ethoxy-3-[4-[2-[4-[2-ethoxy-3-(2-methylprop-2-enoyloxy)propoxy]phenyl]propan-2-yl]phenoxy]propyl] 2-methylprop-2-enoate
BisGMA = Bisphenol A-glycidyl methacrylate

Isomers in the sense of this invention are stereoisomers, including enantiomers.

Inhibitor in the sense of this invention is a polymerization inhibitor.

Trogers' base = 2,8-Dimethyl-6H,12H-5,11-methanodibenzo[b,f][1,5]diazocine

Example 1 describes preparation of dental compositions A, B, C and D comprising different amounts of the at least one polymerizable monomer, the at least one polymerization initiator, the at least one polymerization coinitiator and the at least one polymerization inhibitor.

Example 2 describes preparation of a dental composition A, B, C and D as defined above comprising additionally 70 weight% of silanated glass filler Example 3 described preparation of dental composition comprising 0.25 weight% of camphorquinone and different amounts of dibenzylaniline, BHT and DPI.

Example 4 describes measurement of Flexural strength and E-modulus.

Example 5 describes measurement of b-value and opacity.

Example 6 describes measurement of Working time. Working time was measured as sensitivity to ambient light at 9000 lux.

Example 7 describes measurement of Depth of cure.

Example 8 describes measurement of DSC-Integral and DSC-slope. The DSC-Integral was determined at illumination at 1000mW blue light (400-500nm).

### Example 1: Preparation of dental compositions A, B, C and D

Four mixtures comprising BisGMA and TEGDMA in a 70 to 30 wt% ratio, about 0.25 wt% camphorquinone, about 1 wt% of an aromatic benzylamine (either dibenzyl aniline or Troeger's base) and either BHT or no inhibitor (about 0.25 wt%, in the latter case replaced by more monomers) were prepared and stirred for one hour in the dark at 50°C until all compounds are homogeneously mixed (Table 1).

### Example 2: Preparation of dental compositions A, B, C and D with filler

To each of the three liquids A, B, C, or D (30 wt%) (Table 1) was added 70 wt% silanized glass filler and the mixtures were mixed (Hauschild SpeedMixer) five times at 2150 rpm for 2 min and finally once for 1 min at 1500 rpm at 50 mbar.

### Example 3: Preparation of composite matrix liquids based on UDMA-Resin

A monomer mixture from 99.75 wt% UDMA-Resin and 0.25 wt% camphorquinone was prepared and stirred in the dark at 50°C until the complete dissolution. The mixture was splitted in two parts and two different concentrations (1 wt% and 0.5 wt%, respectively) of dibenzylaniline were added. The mixtures were stirred at 50°C until complete dissolution. Each of the mixtures was splitted in two parts and in every second one, 0.75 wt% dimethyl diphenyl iodonium hexafluorophosphate (DPI) was added. The mixtures were stirred at 50°C until complete dissolution. All four mixtures were each splitted in two parts. Two different concentrations of BHT (0.25 wt% and 0.75 wt%, respectively) were added. The 8 mixtures (Table 4) were mixed at 2350 rpm for 6 min (Hauschild SpeedMixer). Four mixtures without the addition of any co-initiator were prepared accordingly for comparison.

### Example 4: Measurement of Flexural strength and E-modulus

The material is filled into a metal mold of approximately 2x2x30 mm which is subsequently covered with foil and manually pressed. The material is photopolymerized for 2 min on each side using a LicuLite light oven with UV-filter. After removal from the mold, excess of material is removed by wet sanding with grit paper 1000. The specimens are conditioned in water at 37°C before they are subjected to a three-point-bending test at a Zwick universal testing machine. The span width is 20 mm, the feeding speed is 1 mm/min. For the determination of the E-modulus, the linear elastic region is used.

### Example 5: Measurement of b-value and opacity

The material is filled into a round mold of 30 mm diameter and 1 mm thickness between two sheets of foil and manually pressed. It is photopolymerized for 2 min on each side using a LicuLite light oven with UV-filter. The color measurement is done at a Datacolor 800. For the measurement of L, a, and b-value, the plates are placed on a white tile with a drop of 1 wt% sodium dodecyl sulfate solution. The result is the average of the measurement at 5 points. The opacity value is obtained in the same fashion but on a black tile.

### Example 6: Measurement of working time

Stability against ambient light or alternatively curing or working time was determined using a Suntester CPS. A droplet of the paste was squeezed between two microscopy slides and put underneath a neutral light filter (ND, D 1,0). The light intensity was adjusted to 9000 Lux. The sample was irradiated in steps of 10 s and checked if the slides can be slided against each other until polymerization. The accumulated time was noted. The average time of multiple experiments was used as working time.

### Example 7: Measurement of Depth of cure

The material is filled into a cylindrical mold of 4 mm diameter and 12 mm height. The mold is covered with foil and manually pressed. It is irradiated with a dental hand lamp (SmartLite Focus) for 10 s. After removal from the mold, unpolymerized material is gently removed from the specimen with a plastic spatula. The height of the remaining cured object is measured and divided by two to obtain the depth of cure.

### Example 8: Measurement of DSC-Integral and DSC-slope

The material is filled into aluminum trays (30 mg) and inserted in a DSC-device (DSC 204 F1 Phoenix Netzsch). The temperature is equilibrated at 25°C and the measurement chamber is purged with nitrogen gas. The sample is irradiated at 1000 mW (OmniCure 2000 with 400-500nm color filter) for 20 seconds and the polymerization heat is monitored. To correct for photothermal effects, the heat of an empty aluminum tray is subtracted. The integral of the evolved heat over time and the maximum slope are the results.

**Table 1: Composition of dental formulations A, B, C, and D.**

| Composition | A | B | C | D |
|---|---|---|---|---|
| BisGMA(weight%) | 68.947 | 69.125 | 68.947 | 69.125 |
| TEGDMA (weight%) | 29.549 | 29.625 | 29.549 | 29.625 |
| CQ (weight%) | 0.251 | 0.25 | 0.251 | 0.25 |
| Aromatic benzylamine | Dibenzylaniline | Dibenzylaniline | Troeger's base | Troeger's base |
| Aromatic benzylamine (weight%) | 1.002 | 1.000 | 1.002 | 1.000 |
| BHT (weight%) | 0.251 | - | 0.251 | - |

**Table 2: Characteristics of the dental compositions of Table 1.**

| Composition | A | B | C | D |
|---|---|---|---|---|
| E-Modulus (MPa) | 2260 | 2250 | 1580 | 2000 |
| Flexural strength (MPa) | 96 | 96 | 69 | 92 |
| b-value | 4.8 | 5.1 | 4.4 | 5.2 |
| Working time (s) | 160 | 130 | More than 240 | More than 240 |
| DSC-Integral (J/g) | 58 | 66 | 36 | 47 |
| DSC-slope (mW/mg/min) | 8.6 | 10.6 | 4.9 | 6.3 |

**Table 3: Characteristics of the dental compositions of Table 1 comprising additionally 70 weight% of silanated glass filler**

| Composition | A | B | C | D |
|---|---|---|---|---|
| E-Modulus (MPa) | 8750 | 8430 | 7030 | 7220 |
| Flexural strength (MPa) | 124 | 114 | 111 | 117 |
| B-value | 1.4 | 1.5 | -0.1 | 0.0 |
| Working time (s) | 90 | 70 | | |
| Opacity (%) | 24.3 | 22.8 | 22.6 | 22.6 |
| Depth of cure (mm) | More than 4.8 | More than 4.8 | 3.4 | 4.1 |
| DSC-Integral (J/g) | 35 | 36 | | |
| DSC-slope (mW/mg/min) | 8.7 | 9.3 | | |

**Table 4: Characteristics of dental compositions comprising UDMA, 0.25 weight% of camphorquinone and the indicated amounts of DPI, dibenzylaniline and BHT (all in weight%).**

| **Dibenzylaniline** | **BHT** | **DPI** | **b-value** | **E-modulus (MPa)** | **Working time (sec)** | **DSC-Integral (J/g)** |
|---|---|---|---|---|---|---|
| 0.50 | 0.25 | - | 3.4 | 1370 | 120 | 79 |
| 0.50 | 0.75 | - | 3.6 | 1500 | 170 | 64 |
| 0.50 | 0.25 | 0.75 | 4.6 | 1380 | 120 | 117 |
| 0.50 | 0.75 | 0.75 | 7.2 | 1250 | 220 | 101 |
| 1.00 | 0.25 | - | 3.3 | 1460 | 100 | 82 |
| 1.00 | 0.75 | - | 3.5 | 1410 | 150 | 72 |
| 1.00 | 0.25 | 0.75 | 4.4 | 1180 | 90 | 125 |
| 1.00 | 0.75 | 0.75 | 5.3 | 1110 | 160 | 112 |
| - | 0.25 | - | 2.5 | Low (not measurable) | More than 240 | 23 |
| - | 0.75 | - | 4.3 | Low (not measurable) | More than 240 | 11 |
| - | 0.25 | 0.75 | 2.5 | Low (not measurable) | More than 240 | 24 |
| - | 0.75 | 0.75 | 3.5 | Low (not measurable) | More than 240 | 17 |

## Claims

1. A polymerizable dental composition comprising a mixture of
(a) at least one polymerizable monomer having a polymerizable carbon-carbon double bond;
(b) an initiator system which is soluble in the mixture, comprising
(b1) a photoinitiator compound, and
(b2) a compound of the following formula (I), or a salt thereof: 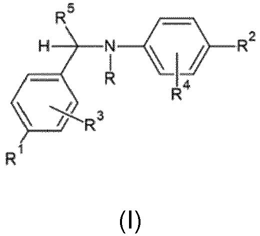 wherein
R¹ and R² , which may be the same or different, independently represent a hydrogen atom, or a C₁₋₆ alkyl group;
R³ and R⁴ which may be the same or different, independently represent a hydrogen atom, or a C₁₋₆ alkyl group, or R³ and R⁴ together represent a single bond, a methylene group, or form together with the carbon atoms to which they are attached an 8- to 10-membered non-aromatic heterocyclic ring, provided that either R³ or R⁴ does not form a ring with R or R⁵;
R⁵ represents a hydrogen atom or a C₁₋₆ alkyl group, or R⁵ forms with either R³ or R⁴, respectively, and together with the carbon atoms to which they are attached a non-aromatic 6-to 10-membered heterocyclic ring, provided that R⁵ does not form a ring with R; and
R represents a hydrogen atom, an optionally substituted phenyl group, or a C₁₋₆ alkyl group which may be substituted by an optionally substituted phenyl group, wherein the substituent on the optionally substituted phenyl group is selected from a C₁₋₆ alkyl group, or
R forms together with either R³, R⁴ or R⁵, respectively, and the carbon atoms to which they are attached a non-aromatic 5- to 10-membered heterocyclic ring which may contain an endocyclic group selected from an amide group, an ester group, an ether group, or an amino group optionally substituted by a C₁₋₆ alkyl group, or
R forms a C₁₋₃ alkylene bridge across the 8 to 10 membered heterocyclic ring formed by R³ and R⁴ and the carbon atoms to which they are attached.

2. The polymerizable dental composition according to claim 1, wherein R⁵ is a hydrogen atom.

3. The polymerizable dental composition according to claim 1 or 2, wherein R³ and R⁴ represent hydrogen atoms.

4. The polymerizable dental composition according to any one of the preceding claims, wherein R is hydrogen atom, or a benzyl group which may be substituted at the aromatic ring by a C₁₋₆ alkyl group, preferably N-benzyl-N-methyl aniline.

5. The polymerizable dental composition according to claim 1, wherein R³ and R⁴ are attached in the ortho position, respectively, and together represent a group of the following formula (II): 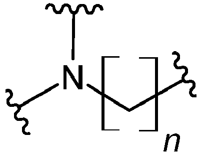 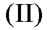 wherein n is an integer of from 1 to 3, and R forms a divalent bridge across the 8 to 10 membered heterocyclic ring formed by R³ and R⁴ and the carbon atoms to which they are attached.

6. The polymerizable dental composition according to claim 1 or 4, wherein R¹ and R² are methyl groups, preferably wherein the compound of formula (I) is Troger's base.

7. The polymerizable dental composition according to claim 1, wherein R³ and R⁴ together represent a methylene group, and R forms together R⁵ and the carbon atoms to which they are attached a non-aromatic 6-membered heterocyclic ring containing an endocyclic group amino group substituted by a C₁₋₆ alkyl group, preferably mianserin.

8. The polymerizable dental composition according to claim 1 or 2, wherein R forms together with R⁴ and the carbon atoms to which they are attached a non-aromatic 6-membered heterocyclic ring containing an endocyclic amide group, preferably 4-benzyl-1,3-dihydroquinoxalin-2-one.

9. The polymerizable dental composition according to any one of the preceding claims, wherein the photoinitiator is camphorquinone.

10. The polymerizable dental composition according to any one of the preceding claims, further comprising a polymerization inhibitor.

11. The polymerizable dental composition according to any one of the preceding claims, further comprising a particulate filler.

12. The polymerizable dental composition according to any one of the preceding claims, further comprising a polymerization accelerator, preferably a diphenyliodonium salt.

13. The polymerizable dental composition according to any one of the preceding claims, further comprising a solvent.

14. The polymerizable dental composition according to any one of the preceding claims, wherein the concentration of the compound of formula (I) is from 0.05 5.00 percent by weight based on the total weight of the polymerizable dental composition; and/or wherein the polymerizable dental composition is selected from a dental composite, a temporary sealing material, a dental cement, a dental adhesive, and a dental fissure sealant.

15. A use of a compound of the following formula (I), or a salt thereof: 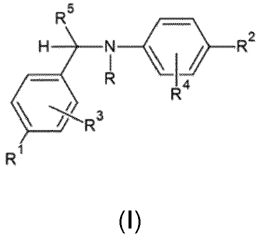 wherein
R¹ and R² which may be the same or different, independently represent a hydrogen atom, or a C₁₋₆ alkyl group;
R³ and R⁴ which may be the same or different, independently represent a hydrogen atom, or a C₁₋₆ alkyl group, or R³ and R⁴ together represent a single bond, a methylene group, or form together with the carbon atoms to which they are attached an 8- to 10-membered non-aromatic heterocyclic ring, provided that either R³ or R⁴ does not form a ring with R or R⁵;
R⁵ represents a hydrogen atom or a C₁₋₆ alkyl group, or R⁵ forms with either R³ or R⁴, respectively, and together with the carbon atoms to which they are attached a non-aromatic 6-to 10-membered heterocyclic ring, provided that R⁵ does not form a ring with R; and
R represents a hydrogen atom, an optionally substituted phenyl group, or a C₁₋₆ alkyl group which may be substituted by an optionally substituted phenyl group, wherein the substituent on the optionally substituted phenyl group is selected from a C₁₋₆ alkyl group, or
R forms together with either R³, R⁴ or R⁵, respectively, and the carbon atoms to which they are attached a non-aromatic 5- to 10-membered heterocyclic ring which may contain an endocyclic group selected from an amide group, an ester group, an ether group, or an amino group optionally substituted by a C₁₋₆ alkyl group, or
R forms a C₁₋₃ alkylene bridge across the 8 to 10 membered heterocyclic ring formed by R³ and R⁴ and the carbon atoms to which they are attached;
in a photocurable dental composition,
preferably wherein the polymerizable dental composition is selected from a dental composite, a temporary sealing material, a dental cement, a dental adhesive and a dental fissure sealant.
